# EUROPEAN PATENT SPECIFICATION

(11) **EP 3 679 008 B1**
(45) Date of publication and mention of the grant of the patent: **22.11.2023**
(21) Application number: 18853891.2
(22) Date of filing: 06.09.2018
(51) Int. Cl.: C07C 47/55, C07C 45/43, C07C 17/20

(54) **AN IMPROVED PROCESS FOR PREPARATION OF TRIFLUOROMETHYLBENZALDEHYDES AND INTERMEDIATES THEREOF**
VERBESSERTES VERFAHREN ZUR HERSTELLUNG VON TRIFLUORMETHYLBENZALDEHYDEN UND ZWISCHENPRODUKTEN DAVON
PROCÉDÉ AMÉLIORÉ POUR LA PRÉPARATION DE TRIFLUOROMÉTHYLBENZALDÉHYDES ET INTERMÉDIAIRES CORRESPONDANTS

(30) Priority: 08.09.2017 IN 201711031774; 08.09.2017 IN 201711031775
(43) Date of publication of application: 15.07.2020
(73) Proprietor: SRF LIMITED, Gurgaon, Haryana 122003 (IN)
(72) Inventor: BOKKA, Deepak, Haryana Gurgaon 122003 (IN); MANICKAM, Ramesh, Haryana Gurgaon 122003 (IN); NAGAPPAN, Arumugam, Haryana Gurgaon 122003 (IN); KUMAR, Kapil, Haryana Gurgaon 122003 (IN); ANAND, Rajdeep, Haryana Gurgaon 122003 (IN)
(74) Representative: AWA Sweden AB
(86) International application number: PCT/IN2018/050578
(87) International publication number: WO 2019/049173

(56) References cited:
- CN-A- 102 516 047
- CN-A- 104 016 840
- CN-A- 104 016 840
- JP-A- 2006 265 133
- FERNANDEZ-BOLANOS J ET AL: "THE SYNTHESIS OF 4,5-BISTRIFLUOROMETHYLBENZIMIDAZOLE", JOURNAL OF THE CHEMICAL SOCIETY,, vol. 1960, no. PART 03, 1 January 1960 (1960-01-01), pages 4003-4010, XP002070715, ISSN: 0368-1769, DOI: 10.1039/JR9600004003

## Description

### FIELD OF THE INVENTION

The present invention provides an improved process for preparation of trifluoromethylbenzaldehyde and intermediates thereof.

### BACKGROUND OF THE INVENTION

Trifluoromethylbenzaldehydes are useful chemical intermediates for the production of pesticides and pharmaceutical products.

Various methods for preparing trifluoromethylbenzaldehydes have been described in the prior art.

U.S. Patent No. 2, 180, 772 describes process for preparation of trifluoromethylbenzaldehydes from trifluoromethylbenzalfluorides by saponification in presence of concentrated sulphuric acid at 80°C to 150°C.

The main disadvantage of this process is that the use of trifluoromethylbenzalfluoride which is prepared from trichloromethylbenzal chloride, this additional step makes the process lengthy and uneconomic.

Journal of Chemical Society 1960, 4003-4010 discloses a process for preparation of o-dichloromethylbenzotrifluoride by reacting o-dichloromethylbenzotrichloride with anhydrous hydrogen fluoride at a temperature of 95-105°C at a pressure of 75 atmosphere.

Japanese Patent Application 2006-265133 discloses a process for preparation of o-dichloromethylbenzotrifluoride by reacting o-dichloromethylbenzotrichloride with hydrogen fluoride. Chinese Patent Application CN104016840 discloses a process for preparation of o-dichloromethylbenzotrifluoride by reacting o-dichloromethylbenzotrichloride with hydrogen fluoride in presence of antimony halide catalyst.

European Patent No. 0077853 describes a process for preparation of meta-trifluoromethyl benzaldehyde from meta-xylene comprising the steps of, chlorinating meta-xylene to form meta-trichloromethylbenzal chloride, reacting the meta-trichloromethylbenzal chloride with anhydrous hydrogen fluoride in the presence of a halogen transfer catalyst to form meta-trifluoromethylbenzal chloride which after isolation subjected to hydrolysis to form the meta-trifluoromethyl benzaldehyde.

The main disadvantage of this process is use of halogen transfer catalyst and use of several techniques like distillation, evaporation for the isolation of meta-trifluoromethyl benzal chloride that makes the process tedious.

Thus there is an urgent need to develop a cost effective, robust and industrially applicable process for preparation of trifluoromethylbenzaldehydes.

The present invention provides a process for preparation of trifluoromethyl benzal chlorides from trichloromethylbenzal chlorides without using any catalyst and hydrolysing trichloromethylbenzal chloride s to trifluoromethylbenzaldehydes.

### OBJECT OF THE INVENTION

The main object of the present invention is to provide a simple, cost effective and industrially applicable process for preparation of trifluoromethylbenzaldehydes and intermediates thereof.

### SUMMARY OF THE INVENTION

The present invention is set out in the appended set of claims. Embodiments of the invention are set out in the dependent claims.

In first aspect, the present invention provides a process for preparation of a compound of formula 1, 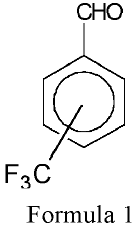 comprising:
a) fluorinating a compound of formula 3 to obtain a compound of formula 2, 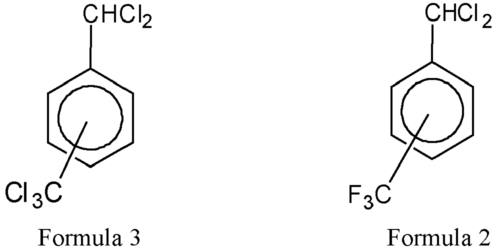
b) flushing an inert gas through the system,
c) contacting the compound of formula 2 with a hydrolysing agent selected from a group consisting of sulphuric acid, sulphur trioxide, nitric acid, phosphoric acid and Lewis acid catalyst, wherein the lewis acid catalyst is selected from a group consisting of zinc chloride, titanium chloride, boron trifluoride, tin chloride and ferric chloride or a mixture thereof, to obtain the compound of formula 1, and
d) isolating the compound of formula 1;

wherein the steps a) to c) are performed without organic solvent;
wherein the step a) is carried out without any catalyst at a pressure range of 1.6 MPa to 2.2 MPa (16 to 22 Kg/cm²) and at a temperature selected in the range from 50 to 150°C; and
wherein the process is carried out in-situ without isolating the compound of formula 2.
Further disclosed is an improved process for preparation of a compound of formula 2, 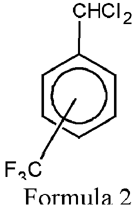 comprising:
a) fluorinating a compound of formula 3 to obtain a compound of formula 2, and 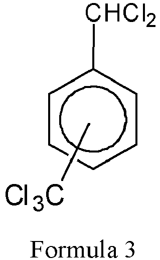
b) isolating the compound of formula 2 obtained from the step a);
wherein the process of step a) is carried out without solvent and a catalyst.

### DETAILED DESCRIPTION OF THE INVENTION

As used herein, the "hydrolysing agent" is selected from a group comprising of sulphuric acid, sulphur trioxide, nitric acid, phosphoric acid, water in presence of Lewis acid catalyst and the like. The Lewis acid catalyst is selected from zinc chloride, titanium chloride, boron trifluoride, tin chloride, ferric chloride and the like. The Lewis acid catalyst is used in catalytic amount and can be recovered during the process. The hydrolysis reaction when carried out in presence of Lewis acid catalyst reduces the cost of the overall process by reducing the effluent load.

The hydrolysis reaction using an acid is performed by adding reaction mixture containing the acid to chilled water.

The hydrolysis reaction of step b) is carried out at a temperature in the range from 30 to 150°C and at a pressure in the range from 3 to 60 Kg/cm² (0.3 to 5.9 MPa).

As used herein, the "inert gas" is selected from a group comprising nitrogen, helium and argon. The step of flushing includes the step of passing inert gas through the system at a flow rate selected in the range of 1 litre per hour to 25 litre per hour which removes hydrochloric acid and reduces the quantities of base required for neutralization. The removal of hydrochloric acid also helps in improving the efficiency and yield of the process.

As used herein, "the step of fluorination" is carried out using hydrogen fluoride in absence of a catalyst and 3 to 20 mole equivalents of hydrogen fluoride gas with respect to the compound of formula 3 can be used. More preferably, 3 to 6 mole equivalents of hydrogen fluoride gas with respect to the compound of formula 3 can be used. The fluorination is performed without a solvent or a catalyst.

The solvent typically includes an organic solvent for the purpose of this invention.

The fluorination is carried out at a temperature in the range from 50 to 150°C, preferably in the range from 60 to 100°C, more preferably in the range from 70 to 90°C and at a pressure in the range from 16 to 22 Kg/cm² (1.6 to 2.2 MPa). It is further disclosed herein that fluorination may be carried out at a temperature in the range from 50 to 150°C, preferably in the range from 60 to 100°C, more preferably in the range from 70 to 90°C and at a pressure in the range from 3 to 60 Kg/cm² (0.3 to 5.9 MPa), preferably in the range from 10 to 30 Kg/cm² (0.9 to 2.9 MPa), more preferably in the range from 16 to 22 Kg/cm² (1.6 to 2.2 MPa).

As used herein, "the step of isolation" is carried out using techniques known in the art for example extraction, decantation, filtration, distillation, evaporation, column chromatography and layer separation or combination thereof.

In an embodiment of first aspect, the present invention provides an in-situ process for preparation of 3-trifluoromethylbenzaldehyde comprising:
a) fluorinating 3-trichloromethylbenzal chloride to obtain 3-trifluoromethylbenzal chloride,
b) flushing an inert gas in the reactor,
c) contacting 3-trifluoromethylbenzal chloride with a hydrolysing agent, and
d) isolating 3-trifluoromethylbenzaldehyde from step c);

wherein the steps a) to c) are performed without solvent;
wherein step a) is carried out without using any catalyst; and
wherein the process is carried out in-situ without isolating the compound of formula 2.

Further disclosed is an improved process for preparation of 3-trifluoromethylbenzal chloride comprising:
a) fluorinating 3-trichloromethylbenzal chloride to obtain trifluoromethylbenzal chloride, and
b) isolating trifluoromethylbenzal chloride from the step a);
wherein the process of step a) is carried out without solvent and a catalyst.

The compound of formula 3 which is used as a starting material may be prepared by any method known in the prior art or may be obtained commercially.

In an embodiment, the preparation of compound of formula 1 from compound of formula 3 is carried out in-situ without isolation of the compound of formula 2, which leads to reduction in process steps and process time and also prevents unwanted exposure of intermediates to the environment and eliminates the requirement of additional isolation steps which leads to a simple, cost effective and industrially applicable process.

Both the fluorination and the hydrolysis reaction is carried out in solvent free conditions.

In a preferred embodiment, the meta-trichloromethylbenzal chloride is charged in an autoclave. The hydrogen fluoride gas is then charged into the autoclave. The reaction mass is heated and stirred at 80°C and at a pressure in the range of 16-22 kg/cm² (1.6-2.2 MPa). The pressure range is maintained by venting off hydrogen chloride gas eventually. The reaction progress is monitored by gas chromatography. After completion of the reaction, reaction temperature is brought to 30°C and hydrogen fluoride gas is vented off. The reaction mass is then flushed with nitrogen to remove excess hydrogen chloride gas and hydrogen fluoride gas. The crude product thus obtained is distilled to obtain pure meta-trifluoromethylbenzal chloride.

While the present invention has been described in terms of its specific embodiments, certain modifications and equivalents will be apparent to those skilled in the art.

The following example is given by way of illustration and therefore should not be construed to limit the scope of the present invention.

### EXAMPLES

### EXAMPLE-1: Preparation of meta-trifluoromethylbenzal chloride.

Meta-trichloromethylbenzal chloride (500 g) was charged in an autoclave. The system was vacuumized. The hydrogen fluoride gas (162 g) was charged into the autoclave. The reaction mass was heated and stirred at 80°C and pressure was maintained at 16 to 22 kg/cm² (1.6 MPa to 2.2 MPa) by venting off hydrogen chloride gas. The reaction progress was monitored by gas chromatography. After completion of the reaction, reaction temperature was brought to 30°C and hydrogen fluoride gas was vented off . The reaction mass was flushed with nitrogen to remove excess hydrogen chloride gas and hydrogen fluoride gas. The crude product thus obtained was distilled to obtain titled compound.
Yield: 90%
Purity: 98.5% (by gas chromatography).

### EXAMPLE-2: Preparation of meta-trifluoromethyl benzaldehyde

Meta-trichloromethylbenzal chloride (500 g) was charged in an autoclave. The system was vacuumized. Hydrogen fluoride (162 g) gas was then charged into the autoclave. The reaction mass was heated to 80°C and pressure was maintained at 16-22 kg/cm² (1.6-2.2 MPa) by venting off hydrogen chloride gas. The reaction progress was monitored by gas chromatography. After completion of the reaction, the temperature was brought to 30°C and the hydrogen fluoride gas was vented off. The reaction mass was flushed with nitrogen to remove excess hydrogen chloride gas and hydrogen fluoride gas. The crude reaction mass (415 g) was taken into the reaction vessel and aqueous solution of sulphuric acid (834 g; 93%) was added to the reaction mass at 80 to 85°C. The reaction was stirred at 80 to 85°C and the reaction progress was monitored by gas chromatography. After completion of the reaction, the reaction mass was added to chilled water (-10 to 5°C) and the crude product was extracted with dichloromethane. The organic layer thus obtained was neutralized with aqueous sodium bicarbonate solution. The crude product (310 g) thus obtained was distilled under vacuum to get the titled compound.
Yield (%): 76.5
Purity: 99.7% (By Gas Chromatography)

### EXAMPLE-3: Preparation of ortho-trifluoromethyl benzaldehyde

Ortho-trichloromethylbenzal chloride (500 g) was charged in an autoclave. The system was vacuumized. Hydrogen fluoride (180 g) gas was then charged into the autoclave. The reaction mass was heated to 80°C and pressure was maintained at 16-22 kg/cm2 (1.6-2.2 MPa) by venting off hydrogen chloride gas. The reaction progress was monitored by gas chromatography. After completion of the reaction, the temperature was brought to 30°C and the hydrogen fluoride gas was vented off. The reaction mass was flushed with nitrogen to remove excess hydrogen chloride gas and hydrogen fluoride gas. The crude reaction mass (425 g) was taken into the reaction vessel and aqueous solution of sulphuric acid (834 g; 93%) was added to it at 80 to 85°C. The reaction was stirred at 80 to 85°C and the reaction progress was monitored by gas chromatography. After completion of the reaction, the reaction mass was quenched with chilled water (-10 to 5°C) and the crude product was extracted with dichloromethane. The organic layer thus obtained was neutralized with aqueous sodium bicarbonate solution. The crude product (360 g) thus obtained was distilled under vacuum to get the titled compound.
Yield (%): 80.2
Purity: 99.5% (By Gas Chromatography)

### EXAMPLE-4: Preparation of Para-trifluoromethyl benzaldehyde

Para-trichloromethylbenzal chloride (500 g) was charged in an autoclave. The system was vacuumized. Hydrogen fluoride (162 g) gas was then charged into the autoclave. The reaction mass was heated to 80°C and pressure was maintained at 16-22 kg/cm2 (1.6-2.2 MPa) by venting off hydrogen chloride gas. The reaction progress was monitored by gas chromatography. After completion of the reaction, the temperature was brought to 30°C and the hydrogen fluoride gas was vented off. The reaction mass was flushed with nitrogen to remove excess hydrogen chloride gas and hydrogen fluoride gas. The crude reaction mass (418 g) was taken into the reaction vessel and aqueous solution (834 g; 93%) of sulphuric acid was added to it at 80 to 85°C. The reaction was stirred at 80 to 85°C and the reaction progress was monitored by gas chromatography. After completion of the reaction, the reaction mass was quenched with chilled water (-10 to 5°C) and the crude product was extracted with dichloromethane. The organic layer thus obtained was neutralized with aqueous sodium bicarbonate solution. The crude product (350 g) thus obtained was distilled under vacuum to get the titled compound.
Yield (%): 78.5
Purity: 99.8% (By Gas Chromatography)

### EXAMPLE-5: Preparation of meta-trifluoromethyl benzaldehyde

Meta-trichloromethylbenzal chloride (70 g) was charged in an autoclave. The system was vacuumized. Hydrogen fluoride (23 g) gas was then charged into the autoclave. The reaction mass was heated to 80°C and pressure was maintained at 16-22 kg/cm2 (1.6-2.2 MPa) by venting off hydrogen chloride gas. The reaction progress was monitored by gas chromatography. After completion of the reaction, the temperature was brought to 30°C and the hydrogen fluoride gas was vented off. The reaction mass was flushed with nitrogen to remove excess hydrogen chloride gas and hydrogen fluoride gas. The crude reaction mass (51 g) of meta-trifluoromethyl benzal chloride and iron chloride (0.36 g) taken in multineck reactor. This mixture heated to 90-100°C and water (3.52 g) was added for 3 hours maintaining same temperature 90-100°C. After completion of water addition, reaction mass was stirred for an hour at 90-100°C. After completion of reaction, crude as such taken for distillation and obtained 29 g of titled compound.
Yield (%): 84.8
Purity: 99.78% (By Gas Chromatography)

### EXAMPLE-6: Preparation of meta-trifluoromethyl benzaldehyde

Meta-trichloromethylbenzal chloride (70 g) was charged in an autoclave. The system was vacuumized. Hydrogen fluoride (23 g) gas was then charged into the autoclave. The reaction mass was heated to 80°C and pressure was maintained at 16-22 kg/cm2 (1.6-2 MPa) by venting off hydrogen chloride gas. The reaction progress was monitored by gas chromatography. After completion of the reaction, the temperature was brought to 30°C and the hydrogen fluoride gas was vented off. The reaction mass was flushed with nitrogen to remove excess hydrogen chloride gas and hydrogen fluoride gas. The crude reaction mass (51 g) of meta-trifluoromethyl benzal chloride and Zinc chloride (0.3 g) taken in multineck reactor. This mixture heated to 90-100°C and water (3.52 g) was added for 3 hours maintaining same temperature 90-100°C. After completion of water addition, allowed reaction mass was stirred for an hour at 90-100°C. After completion of reaction, crude as such taken for distillation to obtain titled compound
Yield (%): 81.8
Purity: 99.8% (By Gas Chromatography)

### COMPARATIVE EXAMPLE-1: Preparation of meta-trifluoromethyl benzaldehyde (without flushing nitrogen gas)

Meta-trichloromethylbenzal chloride (500 g) was charged in an autoclave. The system was vacuumized. Hydrogen fluoride (162 g) gas was then charged into the autoclave. The reaction mass was heated to 80°C. The reaction progress was monitored by gas chromatography. After completion of the reaction, the temperature was brought to 30°C. The crude reaction mass (415 g) was taken into the reaction vessel and aqueous solution of sulphuric acid (834 g; 93%) was added to the reaction mass at 80 to 85°C. The reaction was stirred at 80 to 85°C and the reaction progress was monitored by gas chromatography. After completion of the reaction, the reaction mass was added to chilled water (-10 to 5°C) and the crude product was extracted with dichloromethane. The organic layer thus obtained was neutralized with aqueous sodium bicarbonate solution. The crude product (310 g) thus obtained was distilled under vacuum to get the titled compound.
Yield (%): 65.5
Purity: 82.3% (By Gas Chromatography)

### COMPARATIVE EXAMPLE-2: Preparation of meta-trifluoromethyl benzaldehyde (without flushing nitrogen)

Meta-trichloromethylbenzal chloride (70 g) was charged in an autoclave. The system was vacuumized. Hydrogen fluoride (23 g) gas was then charged into the autoclave. The reaction mass was heated to 80°C. The reaction progress was monitored by gas chromatography. After completion of the reaction, the temperature was brought to 30°C. The crude reaction mass (51 g) of meta-trifluoromethyl benzal chloride and iron chloride (0.36 g) taken in multineck reactor. This mixture heated to 90-100°C and water (3.52 g) was added for 3 hours maintaining same temperature 90-100°C. After completion of water addition, reaction mass was stirred for an hour at 90-100°C. After completion of reaction, crude as such taken for distillation and obtained 29 g of titled compound.
Yield (%): 84.8
Purity: 84.78% (By Gas Chromatography)

## Claims

1. A process for preparation of compound of formula 1, 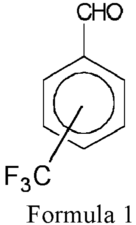 comprising:
a) fluorinating compound of formula 3 to obtain a compound of formula 2, 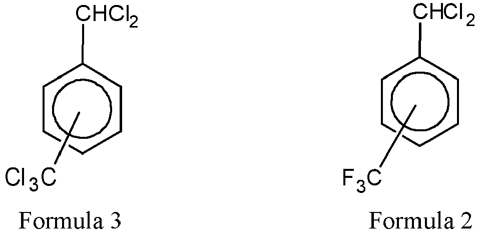
b) flushing an inert gas through the system,
c) contacting the compound of formula 2 with hydrolysing agent selected from a group consisting of sulphuric acid, sulphur trioxide, nitric acid, phosphoric acid and Lewis acid catalyst, wherein the lewis acid catalyst is selected from a group consisting of zinc chloride, titanium chloride, boron trifluoride, tin chloride and ferric chloride or a mixture thereof, to obtain the compound of formula 1, and
d) isolating the compound of formula 1;
wherein the steps a) and c) are performed without organic solvent;
wherein step a) is carried out without any catalyst at a pressure range of 1.6 to 2.2 MPa (16 to 22 Kg/cm2) and at a temperature selected in the range from 50 to 150°C; and
wherein the process is carried out in-situ without isolating the compound of formula 2.

2. The process as claimed in claim 1, wherein fluorination reaction of step a) is carried out using 3 to 20 mole equivalents of hydrogen fluoride gas with respect to the compound of formula 3.

3. The process as claimed in claim 1, wherein the hydrolysis reaction of step b) is carried out at a temperature selected in the range from 30 to 150°C.

4. The process as claimed in claim 1, wherein inert gas is selected from group consisting of nitrogen, helium and argon.

5. The process as claimed in claim 4, wherein inert gas is passed at a flow rate selected in the range of 1 litre per hour to 25 litre per hour.

## Patentansprüche

1. Verfahren zur Herstellung einer Verbindung der Formel 1, 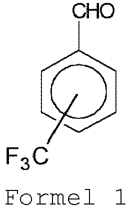 das Folgendes umfasst:
a) Fluorieren einer Verbindung der Formel 3 zum Erhalt einer Verbindung der Formel 2, 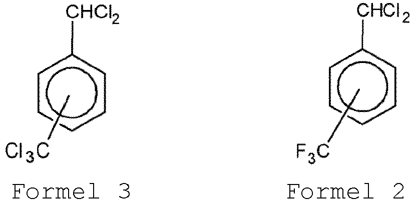
b) Durchspülen des Systems mit einem Inertgas,
c) Inkontaktbringen der Verbindung der Formel 2 mit einem hydrolysierenden Mittel, ausgewählt aus der Gruppe bestehend aus Schwefelsäure, Schwefeltrioxid, Salpetersäure, Phosphorsäure und Lewis-Säure-Katalysator, wobei der Lewis-Säure-Katalysator aus der Gruppe bestehend aus Zinkchlorid, Titanchlorid, Bortrifluorid, Zinnchlorid und Eisen(III)-chlorid oder einer Mischung davon ausgewählt wird, zum Erhalt der Verbindung der Formel 1 und
d) Isolieren der Verbindung der Formel 1;
wobei die Schritte a) und c) ohne organisches Lösungsmittel durchgeführt werden;
wobei Schritt a) ohne Katalysator in einem Druckbereich von 1,6 bis 2,2 MPa (16 bis 22 kg/cm²) und bei einer im Bereich von 50 bis 150 °C gewählten Temperatur durchgeführt wird und
wobei das Verfahren in situ ohne Isolierung der Verbindung der Formel 2 durchgeführt wird.

2. Verfahren nach Anspruch 1, wobei die Fluorierungsreaktion von Schritt a) unter Verwendung von 3 bis 20 Moläquivalenten Fluorwasserstoffgas, bezogen auf die Verbindung der Formel 3, durchgeführt wird.

3. Verfahren nach Anspruch 1, wobei die Hydrolysereaktion von Schritt b) bei einer im Bereich von 30 bis 150 °C gewählten Temperatur durchgeführt wird.

4. Verfahren nach Anspruch 1, wobei das inerte Gas aus der Gruppe bestehend aus Stickstoff, Helium und Argon ausgewählt wird.

5. Verfahren nach Anspruch 4, wobei das inerte Gas mit einer im Bereich von 1 Liter pro Stunde bis 25 Liter pro Stunde gewählten Durchflussrate durchgeleitet wird.

## Revendications

1. Procédé pour la préparation d'un composé de formule 1, 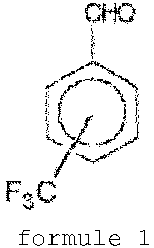 comprenant :
a) la fluoration d'un composé de formule 3 pour obtenir un composé de formule 2, 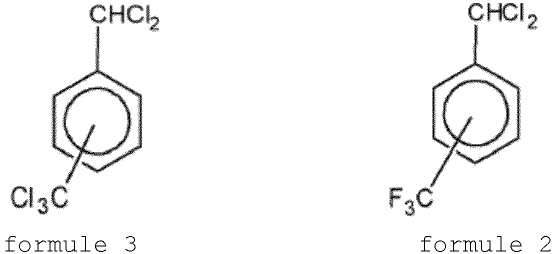
b) la purge d'un gaz inerte à travers le système,
c) la mise en contact du composé de formule 2 avec un agent d'hydrolyse choisi dans un groupe constitué par l'acide sulfurique, le trioxyde de soufre, l'acide nitrique, l'acide phosphorique et un catalyseur de type acide de Lewis, le catalyseur de type acide de Lewis étant choisi dans un groupe constitué par le chlorure de zinc, le chlorure de titane, le trifluorure de bore, le chlorure d'étain et le chlorure ferrique ou un mélange correspondant, pour obtenir le composé de formule 1 et
d) l'isolement du composé de formule 1 ;
les étapes a) et c) étant réalisées sans solvant organique ;
l'étape a) étant mise en œuvre sans aucun catalyseur à une plage de pressions de 1,6 à 2,2 MPa (16 à 22 kg/cm²) et à une température choisie dans la plage de 50 à 150 °C ; et
le procédé étant mis en œuvre *in situ* sans isolement du composé de formule 2.

2. Procédé selon la revendication 1, la réaction de fluoration de l'étape a) étant mise en œuvre en utilisant 3 à 20 équivalents molaires de gaz de fluorure d'hydrogène par rapport au composé de formule 3.

3. Procédé selon la revendication 1, la réaction d'hydrolyse de l'étape b) étant mise en œuvre à une température choisie dans la plage de 30 à 150 °C.

4. Procédé selon la revendication 1, un gaz inerte étant choisi dans un groupe constitué par l'azote, l'hélium et l'argon.

5. Procédé selon la revendication 4, un gaz inerte étant passé à un débit choisi dans la plage de 1 litre par heure à 25 litres par heure.
